## Europäisches Patentamt

## European Patent Office

## Office européen des brevets

(11) Veröffentlichungsnummer: **0 148 369**
**B1**

(12) # EUROPÄISCHE PATENTSCHRIFT

(45) Veröffentlichungstag der Patentschrift:
**10.06.87**

(51) Int. Cl.⁴: **A 61 F 2/26**

(21) Anmeldenummer: **84113688.0**

(22) Anmeldetag: **13.11.84**

(54) Penisprothese.

(30) Priorität: **08.12.83 DE 8335133 U**

(43) Veröffentlichungstag der Anmeldung:
**17.07.85 Patentblatt 85/29**

(45) Bekanntmachung des Hinweises auf die Patenterteilung:
**10.06.87 Patentblatt 87/24**

(84) Benannte Vertragsstaaten:
**DE FR GB SE**

(56) Entgegenhaltungen:
**DE-C-2 646 323**
**DE-U-7 805 284**
**DE-U-8 031 230**
**DE-U-8 335 133**
**US-A-4 151 841**

(73) Patentinhaber: **Koss, Walter, Industriestrasse,
D-6222 Geisenheim/Rhein (DE)**

(72) Erfinder: **Koss, Walter, Industriestrasse, D-6222
Geisenheim/Rhein (DE)**

(74) Vertreter: **Blumbach Weser Bergen Kramer
Zwirner Hoffmann Patentanwälte,
Sonnenbergerstrasse 43, D-6200 Wiesbaden 1 (DE)**

LIBER, STOCKHOLM 1987

EP 0 148 369 B1

## Beschreibung

Die Erfindung betrifft eine Penisprothese in Form eines implantierbaren, biegsamen Stabes mit einem Kern aus mehreren Metalldrähten und mit einer aus implantierbarem Kunststoff bestehenden Außenhülle. Prothesen dieser Art sind bekannt (DE-C-26 46 323, DE-U-78 05 284). Sie werden zur Behandlung der Impotenz bei neurologischen Erkrankungen, Gefäßerkrankungen, Verletzungen und in anderen Fallen jeweils paarweise in die Schwellkörper des Penis inplantiert. Dann kann der Penis in die jeweils gewüschte Form und Position gebogen werden.

Die bekannten Prothesen der erläuterten Art haben sich vielfach bewährt und zwar insbesondere in einer Ausführungsform, bei der die Drähte des Kerns aus hochreinen Silber bestehen. Selbst nach einer sehr großen Zahl von Biegevorgängen härten solche Silberdrähte nicht auf, so daß praktisch keine elastischen Rückstellkräfte auftreten und der Penis in der jeweiligen Biegeform verbleibt.

Eine Penisprothese deren Kern aus einen Bündel von Fasern besteht, ist aus der US-A-4 151 841 bekannt. Der erforderliche Biegewiderstand sowie das Verhindern eines elastischen Rückfederns nach den Biegen wird dabei nicht durch Metalldrähte mit entsprechenden Biegeeigenschaften, sondern durch Zusammenpressen des Faserbündels erzeugt. Dadurch tritt eine hohe Oberflächenreibung zwischen den Fasern auf, wenn diese beim Verbiegen aneinander vorbeigleiten. Nach den Biegen bleibt das Faserbündel in der neuen Form, weil die elastischen Rückstellkräfte die Reibungskräfte nicht überwinden können. Zur Einstellung der Reibungskräfte zwischen den Fasern können diese mit einer Textur ringförmigen Rippen, Einprägungen, einer chemischen Aufrauhung oder auch einer Beschichtung versehen werden.

Biegeversuche an Penisprothesen der eingangs beschriebenen Art mit einem Kern aus Silberdrähten haben gezeigt, daß selbst bei extrem kleinen Biegeradien und großen Biegewinkeln Drahtbrüche erst bei einer sehr großen Zahl von Doppelbiegungen zu erwarten sind.

Selbst bei weiterem Biegen treten keine Drahtenden aus der Prothese aus. Bei implantierten Prothesen finden solche Beanspruchungen in der Praxis zwar nicht statt, weil weder so enge Biegeradien erreicht werden, noch immer an der gleichen Stelle gebogen wird, aber aus Gründen äußerster Vorsicht geht das Bestreben dahin, Prothesen der vorstehend erläuterten Art in Richtung auf eine weiter erhöhte Biegefestigkeit zu verbessern.

Der Erfindung liegt demgemäß die Aufgabe zugrunde, eine implantierbare Penisprothese zu schaffen, deren Drahtkern auch bei einer extrem großen Zahl von Biegungen keine Brüche zeigt.

Zur Lösung der Aufgabe geht die Erfindung aus von einer Penisprothese der eingangs genannten Art und ist dadurch gekennzeichnet, daß wenigstens einige der Drähte einzeln mit einer Hülle aus elastischem Kunststoff mit glatter Oberfläche versehen sind.

Die Drähte, die vorzugsweise wieder aus hochreinem Silber bestehen, liegen dann nicht direkt aneinander, sondern es ist ein Gleiten der glatten Kunststoffoberflächen möglich. Biegeversuche haben ergeben, daß mehr als eine Million Doppelbiegungen ohne Funktionsbeeinträchtigung durchgeführt werden können.

Weiterbildungen der Erfindung sind Gegenstand der Unteransprüche. So wird zweckmäßig die Hülle der Drähte aus Schrumpfschlauch hergestellt. Sie kann jedoch auch durch Umgießen, Umpressen oder Tauchen des Metalldrahtes mit bzw. in Kunststoff hergestellt sein. Darüberhinaus läßt sich die Hülle auch durch Extrudieren erzeugen.

Neben der wesentlich größeren Biegefestigkeit wird auch ein Oberflächenschutz der Drähte erzielt. So hat sich bei Verwendung von Silberdrähten gelegentlich ein schwarzes Verfärben der Oberfläche dann gezeigt, wenn die Prothesen mit Jod oder jodhaltigen Dämpfen in Verbindung kommen. Dann kann ein Diffundieren von Jod durch die meist aus Silicon bestehende Außenhülle stattfinden, wodurch die Schwarzverfärbung ausgelöst wird. Eine dichte Umhüllung der einzelnen Drähte, beispielsweise ein Schrumpfschlauch aus Polytetrafluorethylen (PTFE), verhindert die Diffusion von Jod und damit eine Verfärbung.

Die umhüllten Drähte werden mit Vorteil und in an sich bekannter Weise zur Bildung des Kerns miteinander verdrillt oder verflochten. Dabei können mehrere Einzelstücke eines umhüllten Drahtes verwendet werden, in Weiterbildung der Erfindung besteht aber die Möglichkeit, daß der Kern aus vorzugsweise einem einzigen Stück eines umhüllten Drahtes besteht, der durch mehrfaches, in gleichen Abständen erfolgendes Falten in eine Anzahl paralleler Stränge gebracht ist, die miteinander verdrillt sind. Die durch das Falten erzeugten Schlaufen oder Schlingen an den beiden Enden verbessern das Einbetten in die Außenhülle.

In weiterer Ausbildung der Erfindung läßt sich auch ein Gitter aus sich etwa rechtwinklig kreuzenden, umhüllten Drähten zur Herstellung des Kerns verwenden, indem das Gitter schräg zu den Drähten zusammengerollt wird.

Der Kern selbst kann von der Einbettung in die Außenhülle, die zweckmäßig aus qualitativ hochwertigem, für Implantationen zugelassenen Silicon besteht, mit einer Umhüllung aus Schrumpfschlauch versehen werden. Besonders vorteilhaft ist eine doppelschichtige Ausbildung, bei der über einen Schrumpfschlauch mit verhältnismäßig niedriger Fließtemperatur ein weiterer Schrumpfschlauch mit höherer Fließtemperatur angeordnet ist. Beim Aufschrumpfen kann dann der innere Schrumpfschlauch in die Winkel und Spalten zwischen den umhüllten Drähten eindringen und wird beim Schrumpfen des äußeren Schlauches festgepreßt. Anstelle von zwei getrennten Schrumpfschläuchen läßt sich auch ein Zweischichtenmaterial einsetzen, bei dem die innere Schicht bei einer niedrigeren Temperatur als die äußere Schicht zu fließen beginnt. Die innere Schicht bzw. der innere Schrumpfschlauch kann beispielsweise aus Perflüorethylenpropylen aus (FEP) bestehen und die äußere Schicht bzw. der äußere Schrumpfschlauch aus PTFE. Es kommen auch andere fluorhaltige Polymere in Frage, beispielsweise Ethylen-Tetraflüorethylen (ETFE), Polychlortriflüorethylen (PCTEF), Ethylen-Chlortriflüorethylen (ECTFE) und andere. Die Kristallit-Schmelztemperatur von FEP beträgt z.B. etwa 300°C, die von PTFE etwa 350°C.

Um die Anpassung an die jeweiligen anatomischen Verhältnisse des Patienten sowohl hinsichtlich der Dicke als auch insbesondere der Länge der Prothese zu vereinfachen und gleichzeitig eine umfangreiche und aufwendige Lagerhaltung unterschiedlicher Prothesen zu vermeiden, kann in an sich bekannter Weise (DE-U-80 31 230) vorgesehen sein, daß der Stab in wenigstens zwei Abschnitte unterteilt ist. Der eine Abschnitt besitzt dabei an der Stoßstelle einen Schlauchfortsatz, der über den anderen Abschnitt geschoben werden kann. Durch Kurzen läßt sich dann eine Längenanpassung erreichen.

Nachfolgend wird die Erfindung anhand eines Ausführungsbeispiels in Verbindung mit der Zeichnung beschrieben. Es zeigen:

Fig. 1 die schematische Ansicht einer Penisprothese als Ausführungsbeispiel der Erfindung und

Fig. 2 eine vergrößerte, perspektivische Schnittansicht des Ausführungsbeispiels nach Fig. 1.

Die dargestellte Penisprothese in Form eines Stabes 1 besitzt eine Außenhülle 2 aus implantierbarem Kunststoff, beispielsweise Silicon oder einem anderen Elastomer, und einen eingebetteten Kern 3. Das nach der Implantation distale Ende der Prothese wird durch eine weiche Spitze 4 gebildet, und das andere proximale Ende 5 läuft schlank aus.

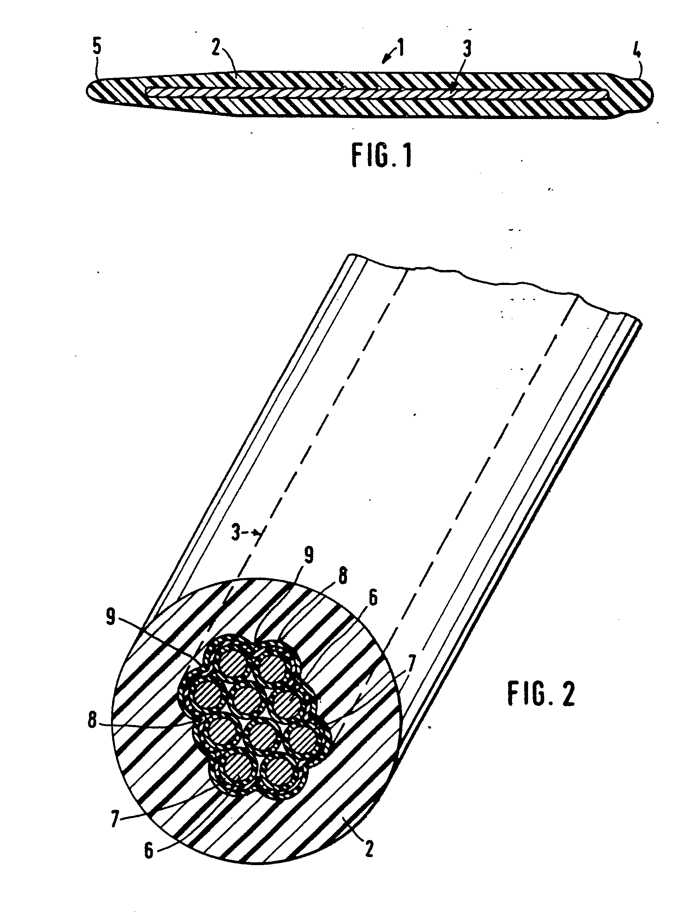

Der Kern 3 weist, wie in Fig. 2 genauer dargestellt, eine Anzahl von Drähten 6 auf, die aus hochreinem Silber bestehen. Jeder einzelne Draht ist in einen Schrumpf schlauch 7 aus PTFE eingeschrumpft, der ursprünglich einen Innendurchmesser von 0,8 mm besitzt. Der eingeschrumpfte Draht 6 mit Schlauch 7 weist dann einen Gesamtdurchmesser von etwa 0,85 mm auf. Zur Bildung des Kerns 3 wurde ein Draht 6, 7 mit etwa der zehnfachen Länge des Kerns 3 neunmal in gleichmäßigen Abständen gefaltet, so daß 10 Stränge oder Abschnitte nebeneinander liegen, wie in Fig. 2 gezeigt. Eine Verdrallung sichert den Zusammenhalt. Die Biegungen oder Schlingen (nicht dargestellt) an den Enden des Kerns 3 verbessern und erleichtern die Einbettung in die Außenhülle 2. Die Drahtabschnitte 6, 7 sind vor der Einbettung in zwei koaxial übereinander liegende Schrumpfschläuche 8, 9 eingeschoben worden, wobei der innere Schlauch 8 aus FEP besteht und im ungeschrumpften Zustandeinen Innendurchmesser von etwa 4,5 mm besitzt. Der äußere Schlauch 9 ist ein dünnwandiger Schrumpfschlauch aus PTFE. Es erfolgt dann ein sorgfältiges Einschrumpfen unter Hitzeeinwirkung, wobei der innere Schlauch 8 soweit erhitzt wird, daß er zu fließen beginnt und in die Zwischenräume zwischen den Drähten 6, 7 eindringt und durch den äußeren Schrumpfschlauch 9 festgepreßt wird.

Die fertige Prothese 1 zeigt ein ausgezeichnetes Biegeverhalten. Sie bleibt in jeder durch Biegung erreichten Stellung praktisch ohne Rückfederung stehen. Dauerbiegeversuche mit einem Biegeradius von etwa 25 mm und einem Doppelbiegewinkel von 2 x 90° mit einer Frequenz von 90 Doppelbiegungen je Minute wurden nach einer Million und in einigen Fällen 1,5 Millionen Doppelbiegungen abgebrochen. Nach dieser Belastung waren die Prothesen in der Dauerbiegezone noch gut abzubiegen und hielten die jeweilige Beugestellung. Auch wenn bei solchen Dauerbiegeversuchen einzelne oder alle Metalldrähte 6 brechen, wird die Funktion nicht beeinträchtigt. Da die Drähte 6 mit ihrer Schrumpfschlauchhülle 7 radial und axial durch die Schläuche 8, 9 fest eingeschrumpft und aneinander gedrückt sind, ändert sich das Biegeverhalten auch nach dem Bruch eines oder mehrerer Drähte 6 kaum. Ein Austreten der Drähte 6 mit einer entsprechenden Verletzungsgefahr ist sicher vermieden. Durch die Einschrumpfung sowohl der Einzeldrähte 6 als auch des Kerns 3 sind die Drähte gegen Eindringen von Fremdstoffen geschützt.

**Patentansprüche**

1. Penisprothese in Form eines implantierbaren, biegsamen Stabes (1) mit einem Kern (3) aus mehreren Metalldrähten (6) und mit einer aus implantierbarem Kunststoff bestehenden Außenhülle (2), dadurch gekennzeichnet, daß wenigstens einige der Drähte (6) einzeln mit einer Hülle (7) aus elastischem Kunststoff mit glatter Oberfläche versehen sind.

2. Penisprothese nach Anspruch 1, dadurch gekennzeichnet, daß die Hülle (7) aus Schrumpfschlauch besteht.

3. Penisprothese nach Anspruch 1, dadurch gekennzeichnet, daß die Hülle (7) durch Umgießen, Umpressen oder Tauchen des Metalldrahtes (6) mit bzw. in Kunstoff hergestellt ist.

4. Penisprothese nach Anspruch 1, dadurch gekennzeichnet, daß die Hülle (7) durch Extrudieren hergestellt ist.

5. Penisprothese nach einem der Ansprüche 1 bis 4, dadurch gekennzeichnet, daß die Drähte (6) aus hochreinem Silber bestehen.

6. Penisprothese nach einem der Ansprüche 1 bis 5, dadurch gekennzeichnet, daß die umhüllten Drähte (6, 7) zur Bildung der Kerns (3) miteinander verseilt oder verflochten sind.

7. Penisprothese nach einem oder Ansprüche 1 bis 6, dadurch gekennzeichnet, daß der Kern (3) aus vorzugsweise einem einzigen Stück eines umhüllten KDrahtes (6, 7) besteht, der durch mehrfaches, in gleichen Abständen erfolgendes Falten in eine Anzahl paralleler Stränge gebracht ist, die miteinander verdrallt sind.

8. Penisprothese nach einem der Ansprüche 1 bis 5, dadurch gekennzeichnet, daß ein Gitter aus sich etwa rechtwinklig kreuzenden, umhüllten Drähten schräg zu den Drähten zu dem Kern (3) zusammengerollt ist.

9. Penisprothese nach einem der Ansprüche 1 bis 8, dadurch gekennzeichnet, daß der Kern (3) mit einer Umhüllung (8, 9) aus Schrumpfschlauch versehen ist.

10. Penisprothese nach Anspruch 9, dadurch gekennzeichnet, daß über einen Schrumpfschlauch (8) mit verhältnismäßig niedriger Fließtemperatur ein Schrumpfschlauch (9) mit höerer Fließtemperatur angeordnet ist.

11. Penisprothese nach Anspruch 9, dadurch gekennzeichnet, daß der Schrumpfschlauch (8, 9) aus einem Zweischichtenmaterial besteht, von dem die innere Schicht (8) bei einer niedrigeren Temperatur als die äußere Schicht (9) zu fließen beginnt.

12. Penisprothese nach einem der vorhergehenden Ansprüche, dadurch gekennzeichnet, daß der Stab (1) in an sich bekannter Weise in wenigstens zwei Abschnitte unterteilt ist.

## Claims

1. A penis prosthesis in the form of an implantable flexiblerod (1) having a core (3) embodied by a number of metal wires (6) and having an outer envelope (2) made of an implantable plastics, characterised in that at least some of the wires (6) have individually an envelope (7) made of a resilient plastics with a smooth surface.

2. A prosthesis according to claim 1, characterised in that the envelope (7) is made of shrinkage sleeving.

3. A prosthesis according to claim 1, characterised in that the envelope (7) is produced by plastics being poured or pressed around the metal wire (6) or by the same being dipped into plastics.

4. A prosthesis according to claim 1, characterised in that the envelope (7) is produced by extrusion.

5. A prosthesis according to any of claims 1-4, characterised in that the wires (6) are made of very pure silver.

6. A prosthesis according to any of claims 1-5, characterised in that the enveloped wires (6, 7) are stranded or braided together to form the core (3).

7. A prosthesis according to any of claims 1 - 6, characterised in that the core (3) is made of preferably a single piece of an enveloped wire (6, 7) brought by repeated equidistant folding into a number of parallel strands which are twisted together.

8. A prosthesis according to any of claims 1 - 5, characterised in that the lattice of enveloped wires which cross one another substantially at right-angles is rolled together towards the core (3) at an inclination to the wires.

9. A prosthesis according to any of claims 1 - 8, characterised in that the core (3) has an envelope (8, 9) of shrink sleeving.

10. A prosthesis according to claim 9, characterised in that shrink sleeving (9) having a relatively high flow temperature is disposed above shrink sleeving (8) having a relatively low flow temperature.

11. A prosthesis according to claim 9, characterised in that the shrink sleeving (8, 9) is made of a two-layer material, the inner layer (8) starting to flow at a lower temperature than the outer layer (9).

12. A prosthesis according to any of the previous claims, characterised in that the rod (1) is in known manner subdivided into at least two parts.

## Revendications

1. Prothèse de pénis sous la forme d'une tige flexible (1) comportant un noyau (3) constitué de plusieurs fils métalliques (6) et d'une enveloppe extérieure (2) réalisée en une matière plastique implantable, caractérisée en ce qu'au moins quelques-uns des fils (6) sont munis individuellement d'une gaine (7) en matière plastique à surface lisse.

2. Prothèse de pénis selon la revendication 1, caractérisée en ce que la gaine (7) est constituée par un tuyau thermorétractable.

3. Prothèse de pénis selon la revendication 1, caractérisée en ce que la gaine (7) est réalisée par enrobage, par coulée, compression ou immersion du fil métallique (6) avec ou dans une matière plastique.

4. Prothèse de pénis selon la revendication 1, caractérisée en ce que la gaine (7) est réalisée par extrusion.

5. Prothèse de pénis selon les revendications 1 à 4, caractérisée en ce que les fils (6) sont réalisés par de l' argent de haute pureté.

6. Prothèse de pénis selon l'une des revendications 1 à 5, caractérisée en ce que les fils gainés (6, 7) sont reliés entre eux par torsadage ou entrelacement pour former le noyau (3).

7. Prothèse de pénis selon l'une des revendications 1 à 6, caractérisée en ce que le noyau (3) est constitué de préférence par un morceau unique d'un fil gainé (6, 7), qui est amené par pliage multiple réalisé à des intervalles égaux, sous la forme d'un nombre de brins parallèles qui sont réunis par torsadage.

8. Prothèse de pénis selon l'une des revendications 1 à 5, caractérisée en ce qu'un réseau formé de fils gainés, se croisant approximativement à angle droit, est enroulé obliquement par rapport aux fils pour former le noyau.

9. Prothèse de pénis selon l'une des revendications 1 à 8, caractérisée en ce que le noyau (3) est muni d'une enveloppe (8, 9) constituée par un tuyau thermorétractable.

10. Prothèse de pénis selon la revendication 9, caractérisée en ce qu'un tuyau thermorétractable (9) possédant une température assez élevée de fluage est emmanché par dessus un tuyau thermorétractable (8) possédant une température de fluage relativement basse.

11. Prothèse de pénis selon la revendication 9, caractérisée en ce que le tuyau thermorétractable (8, 9) est constitué par un matériau à deux couches, dont la couche intérieure (8) commence à fluer à une température plus basse que la couche extérieure (9).

12. Prothèse de pénis selon l'une des revendications précédentes, caractérisée en ce que la tige (1) est subdivisée de façon connue en soi en au moins deux sections.

FIG.1

FIG. 2